# EUROPEAN PATENT APPLICATION

(11) **EP 1 854 895 A1**
(43) Date of publication of application: **14.11.2007**
(21) Application number: 06714886.6
(22) Date of filing: 28.02.2006
(51) Int. Cl.: C12Q 1/68, C12N 15/09

(54) **SIGNAL AMPLIFICATION METHOD**

(30) Priority: 28.02.2005 JP 2005054289
(71) Applicant: Eisai R&D Management Co., Ltd., Tokyo 112-8088 (JP)
(72) Inventor: USUI, Mitsugu, Kouhoku-ku, Yokohama-shi, Kanagawa, 2220 (JP); KANASHIMA, Motohito, 20004 (JP)
(74) Representative: Grünecker, Kinkeldey, Stockmair & Schwanhäusser Anwaltssozietät
(86) International application number: PCT/JP2006/303756
(87) International publication number: WO 2006/093150

(57) **Abstract**

Provided are a signal amplification method of improving signal sensitivity, qualifying properties and handling property in detection of a target gene by using a PALSAR method, a method of detecting a target gene by using the method, and an oligonucleotide probe to be used in the method. A signal amplification method in detection of a target gene using a polymer formed by the use of a plurality of kinds of oligonucleotide probes having complementary base sequence regions capable of hybridizing with each other, including labeling at least one of the plurality of kinds of oligonucleotide probes with acridinium ester for detection.

## Description

### Technical Field

The present invention relates to a signal amplification method in detection of a target gene using a polymer formed by a self-assembly reaction of oligonucleotide probes, a method of detecting a gene using the method, and an oligonucleotide probe to be used in the method.

### Background Art

As a signal amplification method using no enzyme, there have been reported a signal amplification method including: allowing a plurality of kinds of oligonucleotide probes having complementary base sequence regions capable of hybridizing with each other to react, to thereby form a self-assembly substance (polymer) of the probes (hereinafter, referred to as "PALSAR method"), and a method of a detecting a target gene including measuring the polymer by the PALSAR method, to thereby detect a target gene in a sample (Patent Documents 1 to 5, etc.)

Conventional methods of measuring a polymer include: a method of detecting a polymer including forming a polymer, adding an intercalator such as ethidium bromide, and performing fluorescence measurement; and a method of measuring a polymer including forming a polymer using a probe labeled with a fluorescent substance such as Cy3 and performing fluorescence measurement.
[Patent Document 1] JP 3267576 B
[Patent Document 2] JP 3310662 B
[Patent Document 3] WO 02-31192
[Patent Document 4] JP 2002-355081 A
[Patent Document 5] WO 2003-029441
[Patent Document 6] JP 02-503268 A
[Non Patent Document 1] CLINICAL CHEMISTRY, Vol. 35, No. 8, 1588-1594, 1989

### Disclosure of the Invention

### Problems to be solved by the Invention

An object of the present invention is to provide a signal amplification method to improve signal sensitivity, quantitative capability, and operating efficiency in detection of a target gene using the PALSAR method, a method of detecting a target gene using the method, and an oligonucleotide probe to be used in the method.

### Means for solving the Problems

The inventors of the present invention have made extensive studies to improve signal sensitivity in detection of a polymer, and as a result found that use of an oligonucleotide probe labeled with acridinium ester can significantly improve signal sensitivity, quantitative capability, and operating efficiency.
That is, the present invention provides a signal amplification method in detection of a target gene by using a polymer formed by the use of a plurality of kinds of oligonucleotide probes having complementary base sequence regions capable of hybridizing with each other, including labeling at least one of the plurality of kinds of oligonucleotide probes with acridinium ester for detection.

It is preferable that a total of at least two sites of the plurality of kinds of oligonucleotide probes be labeled with acridinium ester.

As the plurality of kinds of oligonucleotide probes, it is preferable to use a pair of oligonucleotide probes including: a first probe that includes three or more of nucleic acid regions including at least a nucleic acid region X, a nucleic acid region Y, and a nucleic acid region Z in the stated order from the 5' end and has a structure represented by the following chemical formula (1); and a second probe that includes three or more of nucleic acid regions including at least a nucleic acid region X', a nucleic acid region Y', and a nucleic acid region Z' in the stated order from the 5' end and has a structure represented by the following chemical formula (2): 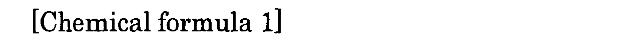 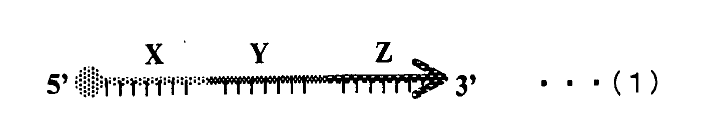 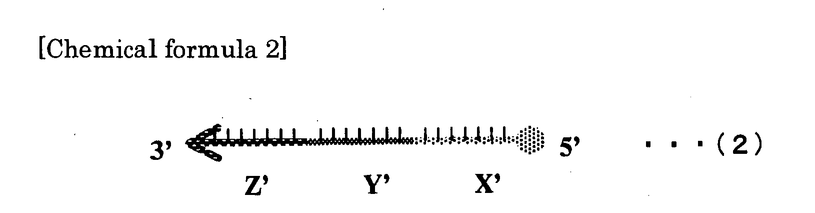 in the chemical formulae (1) and (2), X and X', Y and Y', and Z and Z' are complementary nucleic acid regions capable of hybridizing with each other.

A method of detecting a target gene of the present invention includes detecting a target gene using a signal amplification method of the present invention.

In the method of the present invention, it is preferable that at least one oligonucleotide probe of the oligonucleotide probes has a sequence complementary to a part of the target gene. Further, it is preferable to use an assist probe having regions each complementary to a base sequence of the target gene and to base sequences of the oligonucleotide probes to join the target gene to the polymer.

An oligonucleotide probe of the present invention is a probe to be used in the method of the present invention, which is labeled with acridinium ester.

### Effect of the Invention

According to the present invention, the formation of polymer can be captured directly without influence of steric hindrance, thereby significantly improving signal sensitivity and quantitative capability in detection of a target gene using the PALSAR method. Meanwhile, according to the present invention, a target gene can be detected easily.

### Brief Description of the Drawings

Fig. 1 is a graph showing results of Examples 1 and 2 and Comparative Example 1.
Fig. 2 is a graph showing results of Examples 3 and 4 and Comparative Example 2.

### Best Mode for carrying out the Invention

Hereinafter, embodiments of the present invention will be described with reference to the accompanying drawings, which are for illustrative purposes only, and it will be appreciated that various modifications can be made without departing from the technical idea of the invention.

A signal amplification method of the present invention is a signal amplification method in detection of a target gene using a polymer formed by the use of a plurality of kinds of oligonucleotide probes having complementary base sequence regions capable of hybridizing with each other, which includes labeling at least one of the plurality of kinds of oligonucleotide probes with acridinium ester.

In order to form a polymer from the plurality of kinds of oligonucleotide probes, PALSAR methods described in Patent Documents 1 to 5 may be used.
A first example of the polymer formation is a method of forming a double-stranded self-assembly substance (polymer) using a plurality of pairs of oligonucleotide probes (hereinafter, also referred to as HCPs) including a first probe that includes three or more of nucleic acid regions including at least a nucleic acid region X, a nucleic acid region Y, and a nucleic acid region Z in the stated order from the 5' end and has a structure represented by the following chemical formula (1) and a second probe that includes three or more of nucleic acid regions including at least a nucleic acid region X', a nucleic acid region Y', and a nucleic acid region Z' in the stated order from the 5' end and has a structure represented by the following chemical formula (2) to hybridize the pairs of probes such that they cross in alternation, resulting in self-assembly of the oligonucleotide probes (Patent Documents 1 and 2). 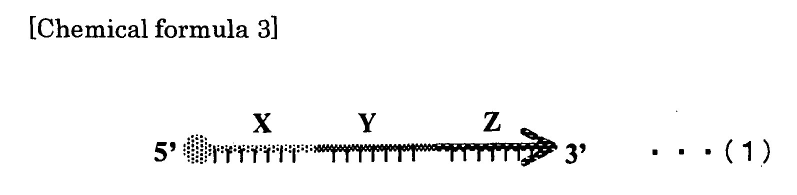 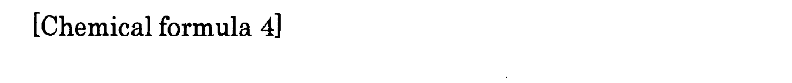 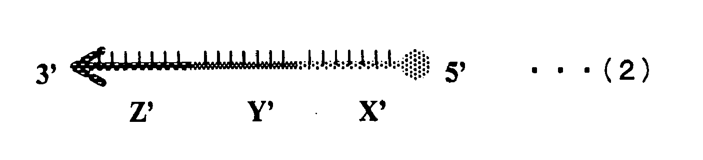 In the chemical formulae (1) and (2), the regions X and X', the regions Y and Y', and the regions Z and Z' are complementary nucleic acid regions capable of hybridizing with each other, and binding of a plurality of pairs of HCPs forms a self-assembly substance represented by the following chemical formula (3).

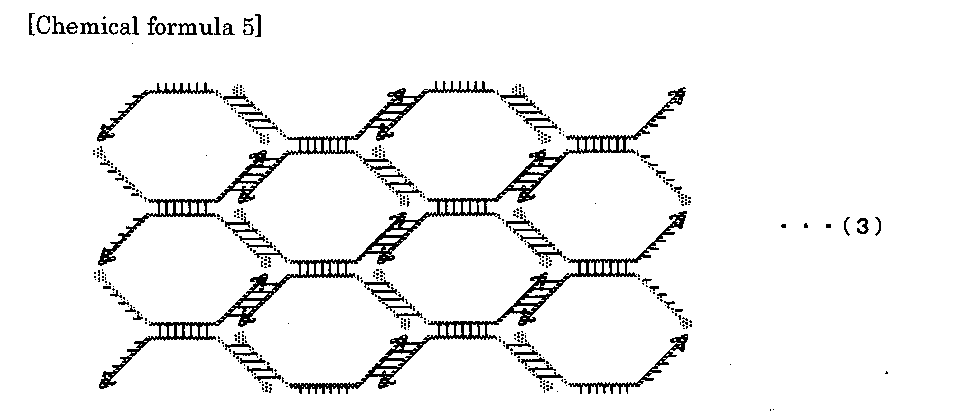

A second example of the polymer formation is a method of forming a self-assembly substance including: providing n groups of dimer forming probes from a first group to a (2n-1)th group (n≧1) in order, in which each group includes a plurality of pairs of dimer forming probes containing a pair of oligonucleotides No. 1 and No. 2, each oligonucleotide having three regions of a 3' side region, a mid-region, and a 5' side region, in which the mid-regions of the oligonucleotides No.1 and No.2 have base sequences complementary to each other, and the 3' side regions and the 5' side regions of the oligonucleotides No.1 and No. 2 have base sequences not complementary to each other and n groups of crosslinking probes, which includes from a second group to a 2n-th group in order, in which each group includes a plurality of pairs of crosslinking probes containing a pair of oligonucleotides No. 1 and No. 2, each oligonucleotide having two regions of a 3' side region and a 5' side region, in which the 3' side regions and the 5' side regions of the oligonucleotides No.1 and No. 2 have base sequences not complementary to each other; designing crosslinking probes so as to have base sequences capable of crosslinking dimmers formed from the dimer forming probes; and hybridizing the probes to forming a self-assembly substance by self-assembly of the oligonucleotides (Patent Document 4).

In the second example, in the case of n=1, there are two combinations of complementary base sequences of dimer probes in the first group and crosslinking probes in the second group. For example, in the case of n=1, the probes may have the following base sequences: the 3' side region of the oligonucleotide No. 1 of the first group and the 3' side region of the oligonucleotide No. 1 of the second group; the 5' side region of the oligonucleotide No. 2 of the first group and the 5' side region of the oligonucleotide No. 2 of the second group; the 3' side region of the oligonucleotide No. 2 of the second group and the 3' side region of the oligonucleotide No. 2 of the first group; and the 5' side region of the oligonucleotide No. 1 of the second group and the 5' side region of the oligonucleotide No. 1 of the first group, have base sequences complementary to each other, respectively.

As another example in the case of n=1, the probes may have the following base sequences: the 3' side region of the oligonucleotide No. 1 of the first group and the 3' side region of the oligonucleotide No. 1 of the second group; the 5' side region of the oligonucleotide No. 2 of the first group and the 5' side region of the oligonucleotide No. 2 of the second group; the 3' side region of the oligonucleotide No. 2 of the first group and the 3' side region of the oligonucleotide No. 2 of the second group; and the 5' side region of the oligonucleotide No. 1 of the first group and the 5' side region of the oligonucleotide No. 1 of the second group, have base sequences complementary to each other, respectively.

In the second example, in the case of n≧2, there are two combinations of complementary base sequences of dimer forming probes in the first, third, ..., (2n-1)th groups and crosslinking probes in the second, fourth, ..., 2n-th groups. For example, in the case of n≧2, the probes may have the following base sequences: the 3' side region of the oligonucleotide No. 1 of the (2n-3)th group and the 3' side region of the oligonucleotide No. 1 of the (2n-2)th group; the 5' side region of the oligonucleotide No. 2 of the (2n-3)th group and the 5' side region of the oligonucleotide No. 2 of the (2n-2)th group; the 3' side region of the oligonucleotide No. 2 of the (2n-2)th group and the 3' side region of the oligonucleotide No. 2 of the (2n-1)th group; the 5' side region of the oligonucleotide No. 1 of the (2n-2)th group and the 5' side region of the oligonucleotide No. 1 of the (2n-1)th group; the 3' side region of the oligonucleotide No. 1 of the last group of the dimer forming probes and the 3' side region of the oligonucleotide No. 1 of the last group of the crosslinking probes; the 5' side region of the oligonucleotide No. 2 of the last group of the dimer forming probes and the 5' side region of the oligonucleotide No. 2 of the last group of the crosslinking probes; the 3' side region of the oligonucleotide No. 2 of the last group of the crosslinking probes and the 3' side region of the oligonucleotide No. 2 of the first group; and the 5' side region of the oligonucleotide No. 1 of the last group of the crosslinking probes and the 5' side region of the oligonucleotide No. 1 of the first group, have base sequences complementary to each other, respectively.

As another example in the case of n≧2, the probes may have the following base sequences: the 3' side region of the oligonucleotide No. 1 of the (2n-3)th group and the 3' side region of the oligonucleotide No. 1 of the (2n-2)th group; the 5' side region of the oligonucleotide No. 2 of the (2n-3)th group and the 5' side region of the oligonucleotide No. 2 of the (2n-2)th group; the 3' side region of the oligonucleotide No. 2 of the (2n-2)th group and the 3' side region of the oligonucleotide No. 2 of the (2n-1)th group; the 5' side region of the oligonucleotide No. 1 of the (2n-2)th group and the 5' side region of the oligonucleotide No. 1 of the (2n-1)th group; the 3' side region of the oligonucleotide No. 1 of the last group of the dimer forming probes and the 3' side region of the oligonucleotide No. 1 of the last group of the crosslinking probes; the 5' side region of the oligonucleotide No. 2 of the last group of the dimer forming probes and the 5' side region of the oligonucleotide No. 1 of the last group of the crosslinking probes; the 3' side region of the oligonucleotide No. 2 of the last group of the crosslinking probes and the 3' side region of the oligonucleotide No. 2 of the first group; and the 5' side region of the oligonucleotide No. 2 of the last group of the crosslinking probes and the 5' side region of the oligonucleotide No. 1 of the first group, have base sequences complementary to each other, respectively.

A third example of the polymer formation is a method including: providing a plurality of groups from a first group to a k-th (k≧2) group in order, in which each group includes a pair of dimer forming probes containing a pair of oligonucleotides No. 1 and No. 2, each oligonucleotide having three regions of a 3' side region, a mid-region and a 5' side region, in which the mid-regions of the oligonucleotides No.1 and No.2 have base sequences complementary to each other, and the 3' side regions and the 5' side regions of the oligonucleotides No.1 and No. 2 have base sequences not complementary to each other, in which (a) the 3' side region of the oligonucleotide No. 1 of the (k-1)th group and the 3' side region of the oligonucleotide No. 2 of the k-th group, (b) the 5' side region of the oligonucleotide No. 2 of the (k-1)th group and the 5' side region of the oligonucleotide No. 1 of the k-th group, (c) the 3' side region of the oligonucleotide No. 1 of the last group and the 3' side region of the oligonucleotide No. 2 of the first group, and (d) the 5' side region of the oligonucleotide No. 2 of the last group and the 5' side region of the oligonucleotide No. 1 of the first group, have base sequences complementary to each other, respectively; and hybridizing a plurality of pairs of dimer forming probes from the first group to the k-th group to form a self-assembly substance by self-assembly of the oligonucleotides (Patent Document 3).

A fourth example of the polymer formation is a method including: providing plural groups from a first group to a k-th (k≧2) group in order, in which each group includes a pair of dimer forming probes containing a pair of oligonucleotides No. 1 and No. 2, each oligonucleotide having three regions of a 3' side region, a mid-region, and a 5' side region, in which the mid-regions of the oligonucleotides No.1 and No.2 have base sequences complementary to each other, and the 3' side regions and the 5' side regions of the oligonucleotides No.1 and No. 2 have base sequences not complementary to each other, in which (a) the 3' side region of the oligonucleotide No. 1 of the (k-1)th group and the 3' side region of the oligonucleotide No. 2 of the k-th group, (b) the 5' side region of the oligonucleotide No. 1 of the (k-1)th group and the 5' side region of the oligonucleotide No. 2 of the k-th group, (c) the 3' side region of the oligonucleotide No. 1 of the last group and the 3' side region of the oligonucleotide No. 2 of the first group, and (d) the 5' side region of the oligonucleotide No. 1 of the last group and the 5' side region of the oligonucleotide No. 2 of the first group, have base sequences complementary to each other, respectively; and hybridizing a plurality of pairs of dimer forming probes from the first group to the k-th group to form a self-assembly substance by self-assembly of the oligonucleotides (Patent Document 3).

In oligonucleotide probes of the present invention, the site and number of labeling with acridinium ester are not particularly limited. Among a plurality of kinds of oligonucleotide probes to be used for forming a polymer, at least one oligonucleotide probe may be labeled with acridinium ester at one or more sites, preferably, at two or more sites. In the case of labeling at two or more sites, one kind of oligonucleotide probe may be labeled at two or more sites, or each of two or more kinds of oligonucleotide probes may be labeled at one or more sites.

For example, in the case of using the HCPs as oligonucleotide probes, a pair of HCPs including an HCP labeled with acridinium ester and an unlabeled HCP, and a pair of HCPs including two HCPs labeled with acridinium ester may be used, but it is preferable to label both HCPs. In this case, labeling sites are not particularly limited, but the sites are preferably symmetrically positioned in hybridizing HCPs. For example, the labeling sites are preferably the 5' end or 3' end of each of HCPs.

A method of labeling an oligonucleotide probe with acridinium ester is not particularly limited, and known methods, for example, methods described in Patent Document 6 and Non-Patent Document 1 may be used. Meanwhile, a method of measuring a polymer formed from oligonucleotide probes labeled with acridinium ester is not particularly limited, and a polymer may be measured by known methods such as methods described in Patent Document 6 and Non-Patent Document 1.

The target gene may be a single-stranded DNA and/or RNA, and a double-stranded DNA and/or RNA. In addition, the target gene may be single nucleotide polymorphisms (SNPs).

Specific examples of a method of detecting a target gene include: a method of detecting a target gene including forming a complex of a target gene and a polymer, and detecting a polymer labeled with acridinium ester; and a method of detecting a target gene including detecting a polymer labeled with acridinium ester using a method of forming a polymer only in the case where a target gene is present.

It is preferable to design the oligonucleotide probes so as to have sequences complementary to a part of the target gene. Meanwhile, in order to join the target gene to the polymer, it is preferable to use an assist probe having regions each complementary to a base sequence of a target gene and a base sequence of an oligonucleotide probe.

The oligonucleotide probes are composed usually of DNA or RNA, but may be nucleic acid analogues. The nucleic acid analogues include, for example, peptide nucleic acid (PNA) and Locked Nucleic Acid (LNA). Further, a pair of oligonucleotide probes is composed usually of the kind of nucleic acids, but, for example, a pair of DNA probe and RNA probe may be used. That is, the kind of nucleic acids in the probes can be selected from DNA, RNA or nucleic acid analogues (such as PNA and LNA). Furthermore, the nucleic acid composition in one probe is not required to consist of only one kind of nucleic acids (e.g., DNA only), and as necessary, for example, a oligonucleotide probe (a chimera probe) composed of DNA and RNA may be usable, which is within the scope of the present invention.

The length of each of complementary base sequence regions in the oligonucleotide probes as the number of bases is at least 5 bases, and is preferably 10 to 100 bases, more preferably 13 to 30 bases. These probes may be synthesized by known methods. For example, a DNA probe may be synthesized using a DNA synthesizer type 394 manufactured by Applied Biosystems Inc. by a phosphoramidite method. In addition, the probes may be synthesized by another method such as a phosphate triester method, an H-phosphonate method, and a thiophosphonate method, but all methods may be used.

In the present invention, the number of oligonucleotide probes to be used is not particularly limited, but may be in a range of 10² to 10¹⁵. The composition and concentration of a reaction buffer are not particularly limited, but a general buffer that is commonly used in amplification of nucleic acids is preferably used. The pH of a reaction buffer may be in a pH range of a buffer that is commonly used, and is preferably in a range of pH 7.0 to 9.0. The temperature condition of a hybridization reaction is also not particularly limited and may be a general temperature condition, but it is preferable to form a partial reaction temperature region in a reaction solution, resulting in a self assembly reaction in the reaction temperature region. The reaction temperature applied in the partial reaction temperature region is 40 to 80°C, preferably 55 to 65°C.

In the present invention, it is preferable to form a self-assembly substance from oligonucleotide probes capable of self-assembly for a target gene captured on a reaction substrate for detection of gene to detect a target gene. The reaction substrate is not particularly limited but is preferably a microplate, a DNA microarray, a magnetic particle, etc.

In the present invention, a sample for measurement of a target gene (DNA or RNA) may be any sample that may contain the nucleic acids. The target gene may be appropriately prepared or isolated from a sample and is not particularly limited. Examples thereof include: samples derived from a living body such as blood, serum, urinary, feces, cerebrospinal fluid, tissue fluid, and cell culture; and samples that may contain or be infected with virus, bacteria, or fungus. Meanwhile, there may be used a nucleic acid such as DNA or RNA obtained by amplifying a target gene in a sample by a known method.

### Examples

Hereinafter, the present invention will be described more specifically by way of Examples, but it will be appreciated that these Examples are for illustrative purposes only and should not be construed as limiting the scope of the invention.

### (Example 1)

As oligonucleotide probes to be used for forming a polymer, a pair of HCPs was used, including HCP-1 (the base sequence represented by SEQ ID NO: 4) labeled at the 5' end with acridinium ester and unlabeled HCP-2 (the base sequence represented by SEQ ID NO: 5). Labeling with acridinium ester was carried out using 200201 Acridinium Protein Labeling Kit (manufactured by Cayman Chemical). The following chemical formula (X) represents a structural formula of an oligonucleotide labeled at the 5' end with acridinium ester.

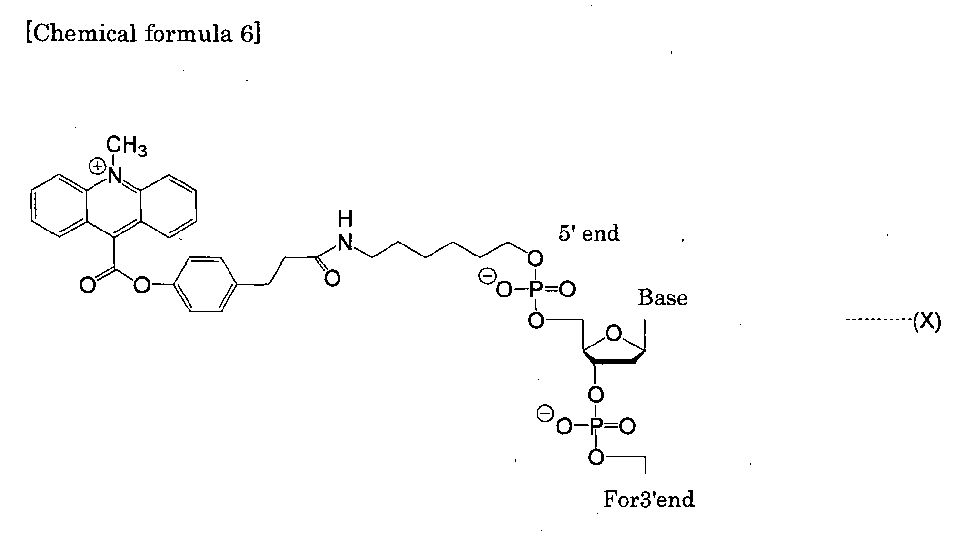

A capture probe having a sequence complementary to rRNA of *Staphylococcus aureu* (the base sequence represented by SEQ ID NO: 1) was immobilized on a microplate.
To the microplate were serially added 50 µL of oligonucleotides (target; the base sequence represented by SEQ ID NO: 2) having the same sequence as rRNA of *Staphylococcus aureu* and diluted to different concentrations (25, 50, 100, 200, 400, 800, or 1,600 fmol/mL) with Tris buffer and 50 µL of a first hybridization solution [4 x SSC, 0.2% SDS, 1% Blocking reagent (manufactured by Roche), 20% formamide, and salmon sperm DNA (10 µg/mL)] containing 24 pmol/mL of an assist probe (the base sequence represented by SEQ ID NO: 3), followed by incubation for two hours under a condition where temperatures of lower and upper parts of the microplate were adjusted to 45°C and 20°C, respectively.

Thereafter, the reaction solutions in the wells were removed, and the wells were washed three times with a washing solution [50 mM Tris, 0.3 M NaCl, 0.01% Triton X-100, pH 7.6], followed by addition of 100 µL of a second hybridization solution [4 x SSC, 0.2% SDS, 1% Blocking reagent (manufactured by Roche)] containing 200 pmoL/mL of a pair of HCPs labeled with acridinium ester. The microplate was incubated for 30 minutes under a condition where temperatures of lower and upper parts of the microplate were adjusted to 55°C and 20°C, respectively.

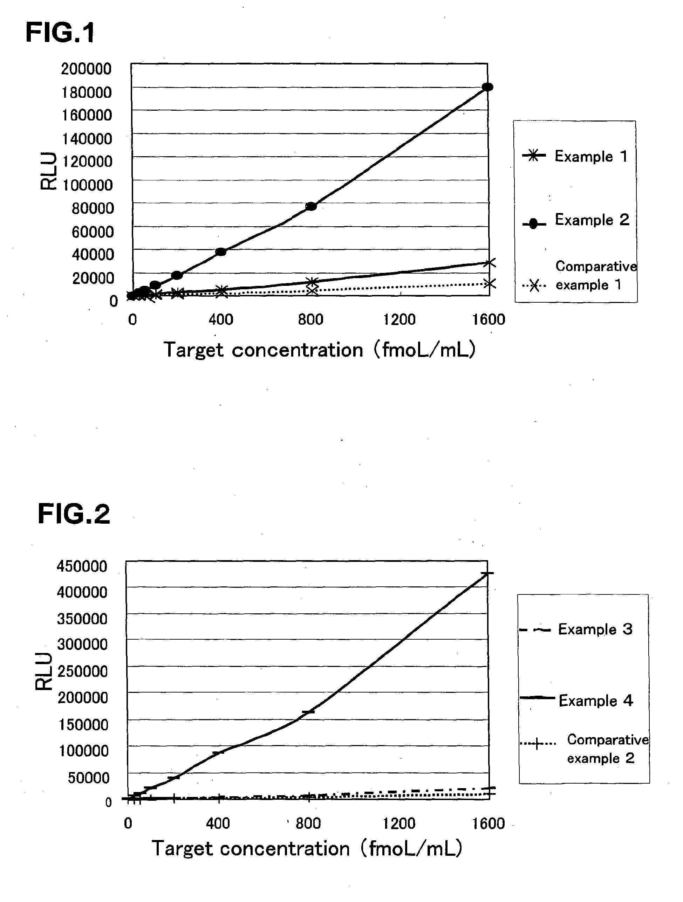

The reaction solutions in the wells were removed, and the wells were washed three times with a washing solution, followed by addition of 50 µL of a luminescence reagent A [0.1% H₂O₂, 0.001 N HNO₃] and 50 µL of a luminescence reagent B [1N NaOH]. Luminescence intensities (RLUs) were immediately measured using a luminometer (manufactured by Berthold, Centro LB-960). The results are shown in Fig. 1 and Table 1.

**[Table 1]**

| Target concentration (fmol/mL) | Example 1 (RLU) | Comparative Example 1 (RLU) | Ratio (Example 1/Comparative Example 1) Example 1) |
|---|---|---|---|
| 1600 | 28766 | 10515 | 2.7 |
| 800 | 12113 | 4414 | 2.7 |
| 400 | 5550 | 2339 | 2.4 |
| 200 | 3022 | 1135 | 2.7 |
| 100 | 1611 | 564 | 2.9 |
| 50 | 711 | 302 | 2.4 |
| 25 | 391 | 152 | 2.6 |
| | | | Average 2.6 |

### (Example 2)

The same experiment as in Example 1 was performed except that, as oligonucleotide probes to be used for forming a polymer, a pair of HCPs was used, including HCP-1 (the base sequence represented by SEQ ID NO: 4) labeled at the 5' end with acridinium ester and HCP-2 (the base sequence represented by SEQ ID NO: 6) labeled at the 5' end with acridinium ester. The results are shown in Fig. 1 and Table 2.

**[Table 2]**

| Target concentration (fmol/mL) | Example 2 (RLU) | Comparative Example 1 (RLU) | Ratio (Example 2/Comparative Example 1) |
|---|---|---|---|
| 1600 | 179395 | 10515 | 17.1 |
| 800 | 77194 | 4414 | 17.5 |
| 400 | 37533 | 2339 | 16.0 |
| 200 | 17127 | 1135 | 15.1 |
| 100 | 9210 | 564 | 16.3 |
| 50 | 4883 | 302 | 16.2 |
| 25 | 2521 | 152 | 16.6 |
| | | | Average 16.4 |

### (Comparative Example 1)

The same method of experiment as in Example 1 was performed except that oligonucleotide probes HCP-1 (the base sequence represented by SEQ ID NO: 4) labeled at the 5' end with acridinium ester only was used. The results are shown in Table 1 and Table 2.

As shown in Tables 1 and 2 and Fig. 1, detection sensitivities of the target gene in Examples 1 and 2 where polymers were formed were improved compared with those in Comparative Example 1 where no polymer was formed. In Example 2 where the oligonucleotide probes labeled at two sites were used, detection sensitivities were particularly improved, and excellent quantitative capabilities were exhibited.

### (Examples 3, 4 and Comparative Example 2)

In Example 3, the same experiment as in Example 1 was performed except that the concentration of HCPs in the second hybridization solution was changed to 1,000 pmol/mL. In Example 4, the same experiment as in Example 2 was performed except that the concentration of HCPs in the second hybridization solution was changed to 1,000 pmol/mL. In Comparative Example 2, the same experiment as in Comparative Example 1 was performed except that the concentration of HCPs in the second hybridization solution was changed to 1,000 pmol/mL. The results are shown in Tables 3, 4 and Fig. 2. As shown in Tables 3, 4 and Fig. 2, detection sensitivities of the target gene in Examples 3 and 4 were improved compared with those in Comparative Example 2. In Example 4, detection sensitivities and quantitative capabilities were particularly remarkably improved.

**[Table 3]**

| Target concentration (fmol/mL) | Example 3 (RLU) | Comparative Example 1 (RLU) | Ratio (Example 3/Comparative Example 2) |
|---|---|---|---|
| 1600 | 19970 | 10028 | 2.0 |
| 800 | 7263 | 3789 | 1.9 |
| 400 | 4153 | 2058 | 2.0 |
| 200 | 1816 | 1135 | 1.6 |
| 100 | 1045 | 588 | 1.8 |
| 50 | 522 | 298 | 1.8 |
| 25 | 450 | 159 | 2.8 |
| | | | Average 2.0 |

**[Table 4]**

| Target concentration (fmol/mL) | Example 4 (RLU) | Comparative Example 2 (RLU) | Ratio (Example 4/Comparative Example 2) |
|---|---|---|---|
| 1600 | 426217 | 10028 | 42.5 |
| 800 | 162630 | 3789 | 42.9 |
| 400 | 87209 | 2058 | 42.4 |
| 200 | 38235 | 1135 | 33.7 |
| 100 | 20035 | 588 | 34.1 |
| 50 | 10458 | 298 | 35.1 |
| 25 | 6946 | 159 | 43.7 |
| | | | Average 39.2 |

### (Examples 5 and 6 and Comparative Example 3)

In Example 5, the same experiment as in Example 3 was performed except that the condition in incubation of the microplate was adjusted to a constant-temperature condition of 45°C by changing the temperature condition of the upper part to the same temperature condition as the lower part. In Example 6, the same experiment as in Example 4 was performed except that the condition in incubation of the microplate was adjusted to a constant-temperature condition of 45°C by changing the temperature condition of the upper part to the same temperature condition as the lower part. In Comparative Example 3, the same experiment as in Comparative Example 2 was performed except that the condition in incubation of the microplate was adjusted to a constant-temperature condition of 45°C by changing the temperature of the upper part to the same temperature as the lower part. The results are shown in Tables 5 and 6.

**[Table 5]**

| Target concentration (fmol/mL) | Example 5 (RLU) | Comparative Example 3 (RLU) | Ratio (Example 5/Comparative Example 3) |
|---|---|---|---|
| 1600 | 12547 | 3824 | 3.3 |
| 800 | 4977 | 1375 | 3.6 |
| 400 | 3519 | 688 | 5.1 |
| 200 | 1834 | 289 | 6.3 |
| | | | Average 4.6 |

**[Table 6]**

| Target concentration (fmol/mL) | Example 6 (RLU) | Comparative Example 3 (RLU) | Ratio (Example 6/Comparative Example 3) |
|---|---|---|---|
| 1600 | 147272 | 3824 | 38.5 |
| 800 | 50683 | 1375 | 36.9 |
| 400 | 32819 | 688 | 47.7 |
| 200 | 14255 | 289 | 49.3 |
| | | | Average 43.1 |

As shown in Tables 5 and 6, detection sensitivities of the target gene in Examples 5 and 6 were improved compared with those in Comparative Example 3. In Example 6, detection sensitivities and quantitative capabilities were particularly remarkably improved.

## Claims

1. A signal amplification method in detection of a target gene using a. polymer formed by the use of a plurality of kinds of oligonucleotide probes having complementary base sequence regions capable of hybridizing with each other, comprising labeling at least one of the plurality of kinds of oligonucleotide probes with acridinium ester for detection.

2. The signal amplification method according to Claim 1, comprising labeling a total of at least two sites of the plurality of kinds of oligonucleotide probes with acridinium ester.

3. The signal amplification method according to Claim 1 or 2, wherein the plurality of kinds of oligonucleotide probes are a pair of oligonucleotide probes including:
a first probe that includes three or more of nucleic acid regions including at least a nucleic acid region X, a nucleic acid region Y, and a nucleic acid region Z in the stated order from the 5' end and has a structure represented by the following chemical formula (1); and
a second probe that includes three or more of nucleic acid regions including at least a nucleic acid region X', a nucleic acid region Y', and a nucleic acid region Z' in the stated order from the 5' end and has a structure represented by the following chemical formula (2); 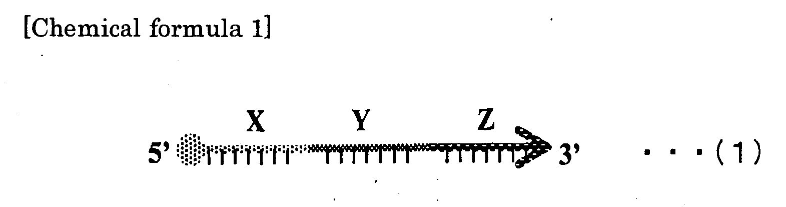 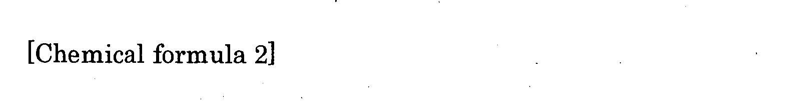 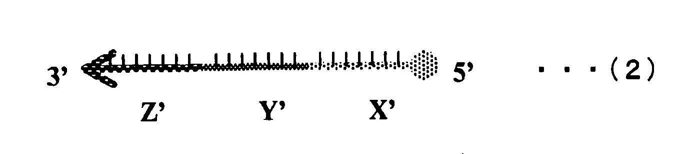 in the chemical formulae (1) and (2), X and X', Y and Y', and Z and Z' are complementary nucleic acid regions capable of hybridizing with each other, respectively.

4. A method of detecting a target gene comprising using a method according to any one of Claims 1 to 3.

5. The method of detecting a target gene according to Claim 4, wherein at least one oligonucleotide probe of the oligonucleotide probes has a sequence complementary to a part of the target gene.

6. The method of detecting a target gene according to Claim 4, comprising using an assist probe having regions each complementary to a base sequence of the target gene and to base sequences of the oligonucleotide probes to join the target gene to the polymer.

7. An oligonucleotide probe, which is labeled with acridinium ester and used in the method according to any one of Claims 1 to 3.
